# EUROPÄISCHE PATENTSCHRIFT

(11) **EP 1 339 853 B1**
(45) Veröffentlichungstag und Bekanntmachung des Hinweises auf die Patenterteilung: **09.08.2006**
(21) Anmeldenummer: 01994705.0
(22) Anmeldetag: 22.11.2001
(51) Int. Cl.: C12N 15/53, C12N 9/02, C12Q 1/66

(54) **ISOLIERTE LUZIFERASEN SOWIE DEREN VERWENDUNG**
ISOLATED LUCIFERASES AND THE USE THEREOF
LUCIFERASES ISOLEES ET LEUR UTILISATION

(30) Priorität: 23.11.2000 DE 10058091
(43) Veröffentlichungstag der Anmeldung: 03.09.2003
(73) Patentinhaber: Bayer HealthCare AG, 51368 Leverkusen (DE)
(72) Erfinder: GOLZ, Stefan, 45326 Essen (DE); KALTHOF, Bernd, 42115 Wuppertal (DE); MARKOVA, Svetlana, Krasnoyarsk, 660036 (RU); FRANK, Ludmila, Krasnoyarsk, 660036 (RU); VYSOTSKI, Eugene, Krasnoyarsk, 660100 (RU)
(86) Internationale Anmeldenummer: PCT/EP2001/013597
(87) Internationale Veröffentlichungsnummer: WO 2002/042470

(56) Entgegenhaltungen:
- WO-A-99/49019
- US-A- 5 604 123
- THOMSON C M ET AL: "The widespread occurrence and tissue distribution of the imidazolopyrazine luciferins." JOURNAL OF BIOLUMINESCENCE AND CHEMILUMINESCENCE, Bd. 12, Nr. 2, 1997, Seiten 87-91, XP001063370 ISSN: 0884-3996
- CAMPBELL A K ET AL: "IMIDAZOLOPYRAZINE BIOLUMINESCENCE IN COPEPODS AND OTHER MARINE ORGANISMS" MARINE BIOLOGY (BERLIN), Bd. 104, Nr. 2, 1990, Seiten 219-225, XP002194746 ISSN: 0025-3162
- INOUYE^A S ET AL: "Secretional luciferase of the luminous shrimp Oplophorus gracilirostris: cDNA cloning of a novel imidazopyrazinone luciferase" FEBS LETTERS, ELSEVIER SCIENCE PUBLISHERS, AMSTERDAM, NL, Bd. 481, Nr. 1, 8. September 2000 (2000-09-08), Seiten 19-25, XP004337549 ISSN: 0014-5793
- SATOSHI INOUYE ET AL: "IMAGING OF LUCIFERASE SECRETION FROM TRANSFORMED CHINESE HAMSTER OVARY CELLS" PROCEEDINGS OF THE NATIONAL ACADEMY OF SCIENCES OF USA, NATIONAL ACADEMY OF SCIENCE. WASHINGTON, US, Bd. 89, Oktober 1992 (1992-10), Seiten 9584-9587, XP002927163 ISSN: 0027-8424
- HASTINGS J W: "Chemistries and colors of bioluminescent reactions: a review" GENE, ELSEVIER BIOMEDICAL PRESS. AMSTERDAM, NL, Bd. 173, Nr. 1, 1996, Seiten 5-11, XP004042846 ISSN: 0378-1119

### SEQUENCE LISTING

<110> Bayer AG
<120> Isolierte Luziferasen sowie deren Verwendung
<130> LeA34790
<150> 10058091.2
   <151> 2000-11-23
<160> 10
<170> PatentIn version 3.1
<210> 1
   <211> 660
   <212> DNA
   <213> Metridia longa
<400> 1
   atggatataa aggttgtctt tactcttgtt ttctcagcat tggttcaggc aaaatcaact      60
   gaattcgatc ctaacattga cattgttggt ttagaaggaa aatttggtat aacaaacctt     120
   gagacggatt tattcacaat atgggagaca atggaggtca tgatcaaagc agatattgca     180
   gatactgata gagccagcaa ctttgttgca actgaaaccg atgctaaccg tggaaaaatg     240
   cctggcaaaa aactgccact ggcagttatc atggaaatgg aagccaatgc tttcaaagct     300
   ggctgcacca ggggatgcct tatctgtctt tcaaaaataa agtgtacagc caaaatgaag     360
   gtgtacattc caggaagatg tcatgattat ggtggtgaca agaaaactgg acaggcagga     420
   atagttggtg caattgttga cattcccgaa atctctggat ttaaggagat ggcacccatg     480
   gaacagttca ttgctcaagt tgaacgttgc gcttcctgca ctactggatg tctcaaaggt     540
   cttgccaatg ttaagtgctc tgaactcctg aagaaatggc tgcctgacag atgtgcaagt     600
   tttgctgaca agattcaaaa agaagttcac aatatcaaag gcatggctgg agatcgttga     660
<210> 2
   <211> 219
   <212> PRT
   <213> Metridia longa
<400> 2
   Met Asp Ile Lys Val Val Phe Thr Leu Val Phe Ser Ala Leu Val Gln
   1 5 10 15
   Ala Lys Ser Thr Glu Phe Asp Pro Asn Ile Asp Ile Val Gly Leu Glu
   20 25 30
   Gly Lys Phe Gly Ile Thr Asn Leu Glu Thr Asp Leu Phe Thr Ile Trp
   35 40 45
   Glu Thr Met Glu Val Met Ile Lys Ala Asp Ile Ala Asp Thr Asp Arg
   50 55 60
   Ala Ser Asn Phe Val Ala Thr Glu Thr Asp Ala Asn Arg Gly Lys Met
   65 70 75 80
   Pro Gly Lys Lys Leu Pro Leu Ala Val Ile Met Glu Met Glu Ala Asn
   85 90 95
   Ala Phe Lys Ala Gly Cys Thr Arg Gly Cys Leu Ile Cys Leu Ser Lys
   100 105 110
   Ile Lys Cys Thr Ala Lys Met Lys Val Tyr Ile Pro Gly Arg Cys His
   115 120 125
   Asp Tyr Gly Gly Asp Lys Lys Thr Gly Gln Ala Gly Ile Val Gly Ala
   130 135 140
   Ile Val Asp Ile Pro Glu Ile Ser Gly Phe Lys Glu Met Ala Pro Met
   145 150 155 160
   Glu Gln Phe Ile Ala Gln Val Asp Arg Cys Ala Ser Cys Thr Thr Gly
   165 170 175
   Cys Leu Lys Gly Leu Ala Asn Val Lys Cys Ser Glu Leu Leu Lys Lys
   180 185 190
   Trp Leu Pro Asp Arg Cys Ala Ser Phe Ala Asp Lys Ile Gln Lys Glu
   195 200 205
   Val His Asn Ile Lys Gly Met Ala Gly Asp Arg
   210 215
<210> 3
   <211> 573
   <212> DNA
   <213> Metridia longa
<400> 3
   atgggagtca aacttatttt tgctgttgtt tgtgtcgcag ttgcccaggc tgccacaatt     60
   caggaaaatt ttgaagacat tgatcttgta gccataggtg gcagctttgc atcagatgtt    120
   gatgctaaca gaggtggaca tggtggacat cctggcaaaa agatgccaaa agaagtactt    180
   atggaaatgg aagccaatgc taaacgagct ggctgccaca ggggttgtct ggtttgtctg    240
   tcacacatca agtgcacagc acaaatgcag aagtttatcc caggaagatg ccatagttat    300
   gcaggagaca aggattctgc tcagggagga attgccggtg gtgccattgt tgatatacct    360
   gaaattgccg gatttaaaga aatgaagccc atggaacagt tcattgctca agttgatctc    420
   tgtgaagatt gcacaactgg atgcctcaaa ggtcttgcca atgttcattg ctctgatctc    480
   ctgaagaagt ggctgccatc aagatgtaag acatttgctt ccaaaattca atctcaagtg    540
   gataccatca aaggtttggc tggagatcgt tga                                 573
<210> 4
   <211> 218
   <212> PRT
   <213> Metridia longa
<400> 4
   Met Asp Met Arg Val Ile Phe Ala Leu Val Phe Ser Ser Leu Val Gln
   1               5                   10                  15
   Ala Lys Ser Thr Glu Phe Asp Pro Asn Ile Asn Ile Val Gly Leu Glu
               20                  25                  30
   Gly Lys Phe Gly Ile Thr Asn Leu Glu Thr Asp Leu Phe Thr Ile Trp
           35                  40                  45
   Glu Thr Met Asp Val Ile Lys Ser Asp Ile Thr Asp Thr Asp Arg Val
       50                  55                  60
   Ser Asn Phe Val Ala Thr Glu Thr Asp Ala Asn Arg Gly Lys Met Pro
   65                  70                  75                  80
   Gly Lys Lys Leu Pro Leu Ala Val Ile Met Glu Met Glu Ala Asn Ala
                   85                  90                  95
   Phe Lys Ala Gly Cys Thr Arg Gly Cys Leu Ile Cys Leu Ser Lys Ile
               100                 105                 110
   Lys Cys Thr Ala Lys Met Lys Val Tyr Ile Pro Gly Arg Cys His Asp
   115 120 125
   Tyr Gly Gly Asp Lys Lys Thr Gly Gln Ala Gly Ile Val Gly Ala Ile
   130 135 140
   Val Asp Ile Pro Glu Ile Ser Gly Phe Lys Glu Met Ala Pro Met Glu
   145 150 155 160
   Gln Phe Ile Ala Gln Val Asp Leu Cys Ala Thr Cys Thr Thr Gly Cys
   165 170 175
   Leu Lys Gly Leu Ala Asn Val Lys Cys Ser Glu Leu Leu Lys Lys Trp
   180 185 190
   Leu Pro Gly Arg Cys Ala Ser Phe Ala Asp Lys Ile Gln Lys Glu Val
   195 200 205
   His Asn Ile Lys Gly Met Ala Gly Asp Arg
   210 215
<210> 5
   <211> 657
   <212> DNA
   <213> Metridia longa
<400> 5
   atggatatga gggttatctt tgctcttgtt ttctcatcat tggttcaggc caaatcaact 60
   gaattcgatc ctaacattaa cattgttggt ttagaaggaa aatttggtat aacaaacctt 120
   gagacggatt tattcacaat atgggagaca atggatgtca tcaaatcaga tattacagat 180
   actgatagag tcagcaactt tgttgcaact gaaaccgatg ctaaccgtgg gaaaatgcct 240
   ggcaaaaaac tgccactggc agttatcatg gaaatggaag ccaatgcttt caaagctggc 300
   tgcaccaggg gatgccttat ctgtctttca aaaataaagt gtacagccaa aatgaaggtg 360
   tacattccag gaagatgtca tgattatggt ggtgacaaga aaactggaca ggcaggaata 420
   gttggtgcaa ttgttgacat tcccgaaatc tctggattta aggagatggc acccatggaa 480
   cagttcattg ctcaagttga tctttgcgct acctgcacta ctggatgtct caaaggtctt 540
   gccaatgtta agtgctctga actcctgaag aaatggctgc ctggcagatg tgcaagtttt 600
   gctgacaaga ttcaaaaaga agttcacaat atcaaaggca tggctggaga tcgttga 657
<210> 6
   <211> 190
   <212> PRT
   <213> Metridia longa
<400> 6
   Met Gly Val Lys Leu Ile Phe Ala Val Val Cys Val Ala Val Ala Gln
   1               5                   10                  15
   Ala Ala Thr Ile Gln Glu Asn Phe Glu Asp Ile Asp Leu Val Ala Ile
               20                  25                  30
   Gly Gly Ser Phe Ala Ser Asp Val Asp Ala Asn Arg Gly Gly His Gly
           35                  40                  45
   Gly His Pro Gly Lys Lys Met Pro Lys Glu Val Leu Met Glu Met Glu
       50                  55                  60
   Ala Asn Ala Lys Arg Ala Gly Cys His Arg Gly Cys Leu Val Cys Leu
   65                  70                  75                  80
   Ser His Ile Lys Cys Thr Ala Gln Met Gln Lys Phe Ile Pro Gly Arg
                   85                  90                  95
   Cys His Ser Tyr Ala Gly Asp Lys Asp Ser Ala Gln Gly Gly Ile Ala
               100                 105                 110
   Gly Gly Ala Ile Val Asp Ile Pro Glu Ile Ala Gly Phe Lys Glu Met
           115                 120                 125
   Lys Pro Met Glu Gln Phe Ile Ala Gln Val Asp Leu Cys Glu Asp Cys
       130                 135                 140
   Thr Thr Gly Cys Leu Lys Gly Leu Ala Asn Val His Cys Ser Asp Leu
   145                 150                 155                 160
   Leu Lys Lys Trp Leu Pro Ser Arg Cys Lys Thr Phe Ala Ser Lys Ile
                   165                 170                 175
   Gln Ser Gln Val Asp Thr Ile Lys Gly Leu Ala Gly Asp Arg
               180                 185                 190
<210> 7
   <211> 657
   <212> DNA
   <213> Metridia longa
<400> 7
   atggatatga aggttatctt tgctcttatt ttctcagcat tggttcaggc caaatcaact 60
   gaattcgatc ctaacattga cattgttggt ttagaaggaa aatttggtat aacaaacctt 120
   gagacggatt tattcacaat atgggagaca atggaggtca tcaaatcaga tattgcagat 180
   actgatagag tcagcaactt tgttgcaact gaaaccgatg ctaaccgtgg gaaaatgcct 240
   ggcaaaaaac tgccactggc agttatcatg gaaatggaag ccaatgcttt caaagctggc 300
   tgcaccaggg gatgccttat ctgtctttca aaaataaagt gtacagccaa aatgaaggtg 360
   tacattccag gaagatgtca tgattatggt ggtgacaaga aaactggaca ggcaggaata 420
   gttggtgcaa ttgttgacat tcccgaaatc tctggattta aggagatggc acccatggaa 480
   cagttcattg ctcaagttga tcgttgcact tcctgcacta ctggatgtct caaaggtctt 540
   gccaatgtta agtgctctga actcctgaag aaatggctgc ctgacagatg tgcaagtttt 600
   gctgacaaga ttcaaaaaga agttcacaat atcaaaggca tggctggaga tcgttga 657
<210> 8
   <211> 630
   <212> DNA
   <213> Metridia longa
<400> 8
   atggatatca aagttctttt tgctcttatt tgcattgcat tggtccaggc caatccaact 60
   gaaaacaatg atcacattaa tattgttggt atagaaggga aatttggtat aacagatctt 120
   gaaacggatt tattcaccat atgggagaca aaccgtatga tcagtacaga taatgaacaa 180
   gccaacacag attctaaccg tggtaaaatg cctgggaaaa aattaccact ggcagtactc 240
   atagaaatgg aagccaatgc ttttaaagct ggctgcacca ggggatgtct tatttgtctt 300
   tctaaaatca agtgtacagc caaaatgaag aagtacattc caggaagatg tcatgattac 360
   ggaggagaca agaaaactgg acaggcaggc atagttggag ctattgttga cattcctgat 420
   atctctggat ttaaagagat gggacccatg gagcagttca ttgctcaagt tgatcgctgc 480
   accgactgca ctactggctg cctcaaaggt cttgccaatg tcaagtgctc tgaactcctc 540
   aaaaaatggc tgccagacag atgtgcaagt tttgctgaca aaattcaaag tgaagtgcac 600
   aacattaagg gccttgctgg agatcgttga 630
<210> 9
   <211> 657
   <212> DNA
   <213> Metridia longa
<400> 9
   atggatataa aggttgtctt tgctcttgtt ttctctgcat tggttcaggc caaatcaact 60
   gaattcgatc ctaacattga cattgttggt ttagaaggaa aatttggtat aacaaacctt 120
   gagacggatt tattcacaat atgggagaca atggaggtca tcaaaacaga tattgcagat 180
   actgatagag ccagaagctt tgttgcaact gaaaccgatg ctaaccgtgg gaaaatgcct 240
   ggcaaaaaac tgccactggc agttatcatg gaaatggaag ccaatgcttt caaagctggc 300
   tgcaccaggg gatgccttat ctgtctttca aaaataaagt gtacagccaa aatgaaggtg 360
   tacattccag gaagatgcca tgattatggt ggtgacaaga aaactggaca ggcaggaata 420
   gtaggtgcaa ttgttgacat tcccgaaatc tctggattta aggagatgga acccatggaa 480
   cagttcattg ctcaagttga tcgttgcgct tcctgcacta ctggatgtct caaaggtctt 540
   gccaatgtta agtgctctga actcctgaag aaatggctgc ctgacagatg tgcaagtttt 600
   gctgacaaga ttcaaaaaga agttcacaat atcaaaggca tggctggaga tcgttga 657
<210> 10
   <211> 657
   <212> DNA
   <213> Metridia longa
<400> 10
   atggatataa aggttgtctt tgctcttgtt ttctctgcat tggttcaggc caaatcaact    60
   gaattcgatc ctaacattga cgttgttggt ttagaaggaa aatttggtat aacaaacctt   120
   gagacggatt tattcacaat atgggagaca atggaggtca tcaaaacaga tattgcagat   180
   actgatagag ccagaaactt tgttgcaact gaaaccgatg ctaaccgtgg gaaaatgcct   240
   ggcaaaaaac tgccactggc agttatcatg gaaatggaag ccaatgcttt caaagctggc   300
   tgcaccaggg gatgccttat ctgtctttca aaaataaagt gtacagccaa aatgaaggtg   360
   tacattccag gaagatgcca tgattatggt ggtgacaaga aaactggaca ggcaggaata   420
   gtaggtgcaa ttgttgacat tcccgaaatc tctggattta aggagatgga acccatggaa   480
   cagttcattg ctcaagttga tcgttgcgct tcctgcaata ctggatgtct caaaggtctt   540
   gccaatgtta agtgctctga actcctgaag aaatggctgc ctgacagatg tgcaagtttt   600
   gctgacaaga ttcaaaaaga agttcacaat atcaaaggca tggctggaga tcgttga      657

## Beschreibung

Die Erfindung betrifft die Nukleotid- und Aminosäuresequenz, sowie die Aktivität und Verwendung der Luziferasen LuAL, Lu164, Lu16, Lu39, Lu45, Lu52 und Lu22.

### Luziferasen

Als Lumineszenz bezeichnet man die Abstrahlung von Photonen im sichtbaren Spektralbereich, wobei diese durch angeregte Emittermoleküle erfolgt. Im Unterschied zur Fluoreszenz wird hierbei die Energie nicht von Außen in Form von Strahlung kürzerer Wellenlänge zugeführt.

Man unterscheidet Chemilumineszenz und Biolumineszenz. Als Chemolumineszenz bezeichnet man eine chemische Reaktion die zu einem angeregten Molekül führt, das selbst leuchtet, wenn die angeregten Elektronen in den Grundzustand zurückkehren. Wird diese Reaktion durch ein Enzym katalysiert, spricht man von Biolumineszenz. Die an der Reaktion beteiligten Enzyme werden generell als Luziferasen bezeichnet

Eine Übersicht über lumineszierende Organismen findet sich bei Hastings et al. 1995.

Bei den Luziferasen handelt es sich um Peroxidasen oder Mono- und Dioxygenasen. Die Enzym-Substrate, die die Ausgangssubstanzen für die lichtemittierenden Produkte bilden, heißen Luciferine. Sie sind von Species zu Species unterschiedlich. Die Quantenausbeute der Systeme liegt zwischen 0,1-0,9 Photonen je umgesetztem Substratmolekül (Idelgaufts, 1993).

Luziferasen lassen sich aufgrund ihrer Herkunft oder ihrer enzymatischen Eigenschaften klassifizieren. Im Folgenden wird ein Überblick über einige Luziferasetypen gegeben:

**Tab. 1 : Bakterielle Luziferasen**

| *Bakterielle Luziferase* | | | | | |
|---|---|---|---|---|---|
| **Genprodukt** | **Organismus** | **Substrat** | **λ** | **Expression** | **Literatur / Patente** |
| Lux-Gene | Vibrio fischerii | FMN, Dodecanal, NADH | 495 nm | cytosolisch | Apley et al., 1985 Gustafson G., US 5196524 |

**Tab. 2 : Coelenterazin-abhängige eukaryotische Luziferasen**

| *Coelenterazine-abhängige eukaryotische Luziferasen* | | | | | |
|---|---|---|---|---|---|
| **Genprodukt** | **Organismus** | **Substrat** | **λ** | **Expression** | **Literatur / Patente** |
| Renilla | Renilla | Coelenterazin | 480 nm | cytosolisch | Mathews et al., 1977 |
| Luziferase | reniformis | | | | Lorenz et al, 1991 |
| | | | | | Lorenz et al. 1996 |
| | | | | | Alan, P; WO 0020619 |
| | | | | | Milton J., US 5418155 |
| | | | | | Roelant C., WO 9938999 |
| Vargula / | Vargula | Vargula- | 460 nm | sekretorisch | Thomspon et al., 1989 |
| Cypridia | hilgendorferii | Luciferin | | | Thompson et al., 1990 |
| Luziferase | | | | | Tora, JP 05064583 |
| | | | | | Tora, JP 08027200 |
| | | | | | Renard et al., |
| | | | | | WO 9520653 |
| Watasemia Luziferase | Watasenia scintillans | Watasemia Luciferin | ? | cytosolisch | Inoue et al., 1976 |
| Olophorus Luziferase | Olophorus gracilirostris | Coelenterazine | 454 | Sekretion | Inouye et al, 2000 |
| Aequorin | Aequoria | Coelenterazin | 470 nm | cytosolisch | Head et al. 2000 |
| | aequoria | (Ca²⁺ aktiviert) | | | Shimomura et al., 2000 |
| | | | | | Jones et al., 1999 |
| | | | | | Kendall et al., 1998 |
| | | | | | Inouye et al., 1985 |
| | | | | | Shimomura et al., 1969 |
| | | | | | Cormier et al., |
| | | | | | US 5798441 |
| | | | | | Cormier et al., |
| | | | | | US 5422266 |
| Obelin | Obelia | Coelenterazin | 470 nm | cytosolisch | Matveev et al., 1999 |
| | | | | | Berestovskaya, 1999 |

**Tab. 3 : Coelenterazin-unabhängige eukaryotische Luziferasen**

| *Coelenterazine-unabhängige eukaryotische Luciferase* | | | | | |
|---|---|---|---|---|---|
| **Genprodukt** | **Organis mus** | **Substrat** | **λ** | **Expression** | **Literatur** |
| Firefly | Photinus | Firefly- | 550 nm | cytosolisch | Webster et al., 1980 |
| Luziferase | pyralis | Luciferin, | | | Gould et al., 1988 |
| | | ATP | | | Sala-Newby et al, 1992 |
| | | | | | Bonin et al., 1994 |
| | | | | | Sherf B., US 5670356 |
| | | | | | KIKK, JP 09187281 |

Luziferasen lassen sich ebenfalls durch ihre Substratspezifität voneinander unterscheiden. Zu den wichtigsten Substraten gehören Coelenterazine (Jones et al., 1999) und Luciferin, sowie Derivate beider Substanzen. Im Folgenden werden die Substrate und ihr Umsatz durch Luziferasen schematisch dargestellt :

### Luziferasesubstrate

Im Folgenden werden einige Luziferasesubstrate und deren Umsatz beispielhaft dargestellt. Alle hier dargestellten Substrate werden unter Freisetzung von Licht und Kohlendioxid (CO₂) und Verbauch von Sauerstoff (O₂) enzymatisch umgesetzt. Die Abhängigkeit der Reaktion von Cofaktoren oder Energieträgern (z.B. ATP für Firefly Luziferase) ist enzymspezifisch.

### Coelenterazin

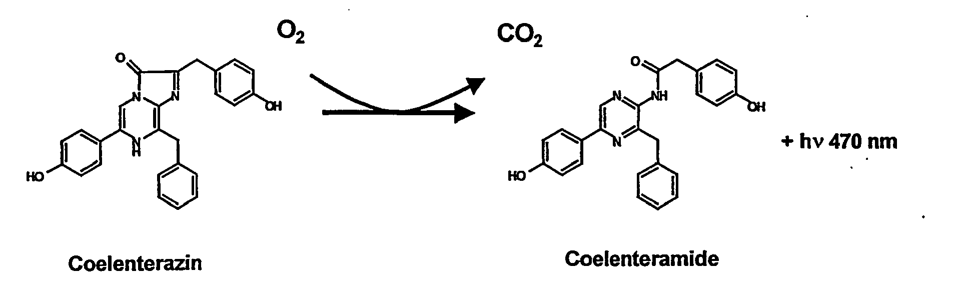

Umsatz von Coelenterazin zu Coelenteramide durch Aequorin unter Emission von Licht der Wellenlänge 470 nm.

### Luciferin

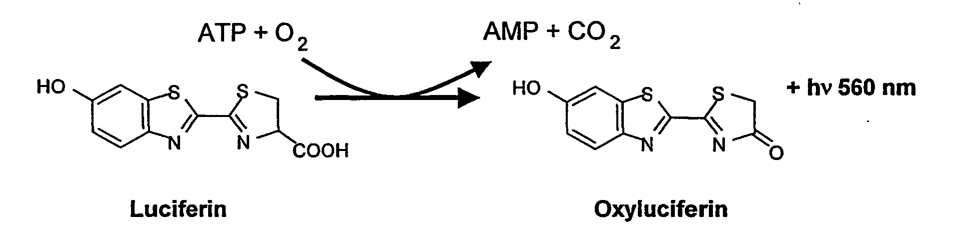

Umsatz von Luciferin zu Oxyluciferin durch Firefly Luciferase unter Emission von Licht der Wellenlänge 560 nm.

### Vargula-Luciferin

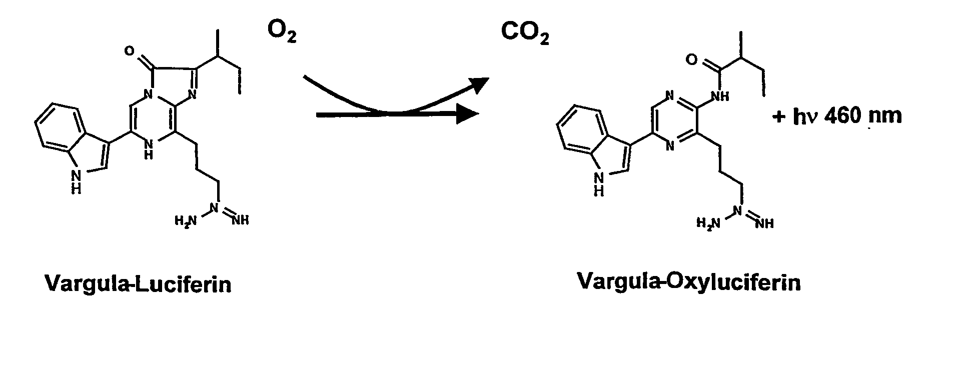

Umsatz von Vargula-Luciferin zu Vargula-Oxyluciferin durch Vargula-Luciferase unter Emission von Licht der Wellenlänge 460 nm.

### Reportersysteme

Als Reporter- oder Indikatorgen bezeichnet man generell Gene, deren Genprodukte sich mit Hilfe einfacher biochemischer oder histochemischer Methoden leicht nachweisen lassen. Man unterscheidet mindestens 2 Typen von Reportergenen.
1. Resistenzgene. Als Resistenzgene werden Gene bezeichnet, deren Expression einer Zelle die Resistenz gegen Antibiotika oder andere Substanzen verleiht, deren Anwesenheit im Wachstumsmedium zum Zelltod führt, wenn das Resistenzgen fehlt.
2. Reportergen. Die Produkte von Reportergenen werden in der Gentechnologie als fusionierte oder unfusionierte Indikatoren verwendet. Zu den gebräuchlichsten Reportergenen gehört die beta-Galaktosidase (Alam et al., 1990), alkalische Phosphatase (Yang et al., 1997; Cullen et al., 1992), Luciferasen und andere Photoproteine (Shinomura, 1985; Phillips GN, 1997; Snowdowne et al., 1984).

### Einordung der Spezies Metridia longa

### Arthropoda →Crustacea →Copepoda

Die Spezies Metridia longa gehört zu den Crustacea, den Krebsen, speziell den Copepoda oder Zooplankton.

### Isolierung der cDNA

Zur Untersuchung der biolumineszenten Aktivität der Spezies Metridia longa wurden Exemplare im Weißen Meer (Biologische Station Kartesh, Russland) gefangen und in flüssigem Stickstoff gelagert. Zur Erstellung von cDNA-Bibliotheken von Metridia longa wurde die RNA nach der Methode von Krieg (Krieg et al., 1996) durch Isothiocyanat isoliert.

Zur Herstellung der cDNA wurde RT-PCR durchgeführt. Hierzu wurden 10 µg RNA mit Reverser Transkriptase (Superscribt Gold II) nach folgendem Schema inkubiert:

| | | | | |
|---|---|---|---|---|
| PCR | 1. | 30 | Sekunden | 95°C |
| | 2. | 6 | Minuten | 68°C |
| | 3. | 10 | Sekunden | 95°C |
| | 4. | 6 | Minuten | 68°C |
| | 17 | Zyklen von Schritt 4 nach Schritt | | 3 |

Die Reaktionsprodukte wurden zur Inaktivierung der Polymerase für 30 Minuten bei 37°C mit Proteinase K inkubiert und die cDNA mit Ethanol präzipitiert. Die cDNA wurde in Wasser gelöst und mit SfiI für eine Stunde bei 37°C inkubiert. Die Reaktionsprodukte wurden zur Abtrennung kleiner Fragmente gelfiltriert. Die fraktionierte cDNA wurde anschließend in den SfiI geschnittenen und dephosphorilierten λTriplEx2 Vector ligiert. Zur Herstellung einer λ-Phagen Expressionsbank wurden die klonierten cDNA-Fragmente anschließend durch das in vitro Verpackungssystem SMART cDNA Library Construction Kits (Clontech) in λ-Phagen verpackt.

Die Identifizierung der rekombinaten Phagen, die eine cDNA Insertion mit potentieller Expression von Colenterazin abhängigen Luziferasen enthielten, wurde ein "library screening" durchgeführt.

Hierzu wurden Bakterienrasen aus E. coli XL1-Blue auf 90 mm Kulturschalen plattiert und für 10 Stunden bei 37°C inkubiert. Anschließend erfolgte die Infektion mit 2500 Phagen pro Kulturschale und eine Inkubationsphase von 8 Stunden bei 37°C zur Plaquebildung. Zur Aushärtung des Weichagars wurden die Kulturschalen anschließend für eine Stunde bei 4°C gelagert.

Zur Durchführung einer Replikaplattierung wurden Nitrozellulosemembranen mit E. coli XL1-Blue Suspensionen getränkt und getrocknet. Die trockenen Membranen wurden für 60 Sekunden auf die Phagenplaques und anschließend auf frische Agarplatten gelegt. Anschließend wurden die Agarplatten für 2 Stunden bei 37°C inkubiert und 4 ml SOB-Medium (+ 10 mM MgSO4, 0,2 % Maltose) zugegeben. Der Bakterienrasen wurde abgelöst, in LB-Medium (+ 20 mM IPTG) resuspendiert und für eine Stunde bei 37°C inkubiert. Die Bakterien wurden durch Zentrifugation geerntet, durch Ultraschall aufgeschlossen und die Biolumineszenzaktivität nach Zugabe von Coelenterazin im Luminometer bestimmt.

Kulturplatten mit positivem Biolumineszenzsignal wurden in Sektoren gegliedert und eine erneute Replikaplattierung durchgeführt. Die Replikaplattierung wurde bis zur Identifizierung von aktiven Einzelplaques weitergeführt. Zur Subklonierung der cDNA-Insertionen der Phagen positiver Plaques erfolgte in den pTriplEX2 Vektor in E. coli BM25.8 nach Herstellerprotokoll für das SMART cDNA library construction kit. Die mit pTriplEx2-cDNA transfierten E. coli wurden in LB Medium mit einer Ampicillinkonzentration von 100 µg/ml über Nacht bei 37°C inkubiert. Zur Überexpression wurde die über Nacht Kultur 1:150 mit LB Medium verdünnt und für 1 Stunde bei 37°C inkubiert. Anschließend erfolgte die Induktion durch die Zugabe von IPTG (Isopropylthiogalaktosid) bis zu einer Endkonzentration von 20 mM. Die induzierte Kultur wurde für 1 Stunde bei 37°C inkubiert und die Bakterien durch Zentrifugation geerntet. Der Zellaufschluß erfolgte durch Ultraschall in 0,5 ml SM Puffer. Die Chemolumineszenz wurde nach der Zugabe von 10 µl Coelenterazin (10⁻⁴ M in Methanol) im Luminometer gemessen.

Es wurden drei Luziferasen identifiziert, die eine Coelenterazin-abhängige Luziferaseaktivität aufwiesen. Die Luziferasen wurden als Lu164, LuAL und Lu22 bezeichnet. Im Folgenden werden die Luziferasen im einzelnen dargestellt.

Die Erfindung betrifft Nukleinsäurtmoleküle, welche die in SEQ ID NO:2, SEQ ID NO: 4 oder SEQ ID NO:6 gezeigte Poptidsequenz kodieren oder mindestens 90% Identität zu diesen Nukleinsäuren aufweisen, und wobei die Nukleinsäuren Peptide kodieren, welche sekretierte Luziferasen sind. Des weiteren betrifft die Erfindung Nukleinsäuremoleküle, welche ein Nukleinsäuremolekül wir das gestellt in SEQ ID NO:1, SEQ ID NO:3, SEQ ID NO:5, SEQ ID NO:7, SEQ ID NO:8, SEQ ID NO: 9, oder SEQ ID NO:10 umfassen, wobei die Nukleinsäuren Luziferasen kodieren. Die Luziferasen eignen sich als Reportergene für zelluläre Systeme speziell für Rezeptoren, für Ionenkanäle, für Transporter, für Transkriptionsfaktoren oder für induzierbare Systeme.

Verwendbar sind die Luziferasen in bakteriellen Sytemen z.B. zur Titerbestimmung oder als Substrate für biochemische Systeme speziell für Proteinasen.

Eingesetzt können die Luziferasen auch als Reporterenzyme gekoppelt an Antikörper oder andere Proteine z.B. für ELISA, für Immunohistochemie oder für Western-Blot.

Einsetzbar sind die Luziferasen in BRET-Systemen (Bioluminescence Resonance Energy Transfer).

Die Luziferasen eignen sich auch als Fusionsprotein für confocale Mikroskopie oder zur Analyse von Protein-Protein-Wechselwirkung.

Verwendbar sind die Luziferasen als Reporterenzym gekoppelt an Biotin, NHS, CN-Br oder andere Kopplungsvermittler z.B. für ELISA oder zur Immobilisierung.

Weiterhin verwendbar sind die Luziferasen als Reporterenzym gekoppelt an DNA- oder RNA-Oligonukleotiden z.B. für Northern- und Southern-Blot oder für realtime PCR.

Die Erfindung betrifft auch die Reinigung der Luziferasen als wildtyp oder Tag-Protein sowie die Verwendung der Luciferasen in in-vitro Translationssystemen.

### Nukleotid- und Aminosäuresequenzen

### LuAL

Die Luziferase LuAL ist ein Protein mit einem Molekulargewicht von 23,7 kDa und einem isolelektrischem Punkt von 8,32. Die kodierende Nukleotidsequenz ist : daraus ergibt sich eine Aminosäuresequenz von: und eine Aminosäurezusammensetzung von:

| | | | | | | | |
|---|---|---|---|---|---|---|---|
| Ala: 18 | (8.3%) | Cys: 10 | (4.6%) | Asp: 14 | (6.4%) | Glu: 12 | (5.5%) |
| Phe: 10 | (4.6%) | Gly: 19 | (8.7%) | His: 2 | (0.9%) | Ile: 18 | (8.3%) |
| Lys: 21 | (9.6%) | Leu: 15 | (6.9%) | Met: 10 | (4.6%) | Asn: 8 | (3.7%) |
| Pro: 7 | (3.2%) | Gln: 5 | (2.3%) | Arg: 7 | (3.2%) | Ser: 9 | (4.1%) |
| Thr: 15 | (6.9%) | Val: 14 | (6.4%) | Trp: 2 | (0.9%) | Tyr: 2 | (0.9%) |

### Lu164

Die Luziferase Lu164 ist ein Protein mit einem Molekulargewicht von 23,8 kDa und einem isolelektrischem Punkt von 7,81. Die kodierende Nukleotidsequenz ist: daraus ergibt sich eine Aminosäuresequenz von: und eine Aminosäurezusammensetzung von:

| | | | | | | | |
|---|---|---|---|---|---|---|---|
| Ala: 21 | ( 9.6%) | Cys: 10 | (4.6%) | Asp: 15 | (6.8%) | Glu: 13 | (5.9%) |
| Phe: 10 | ( 4.6%) | Gly: 18 | (8.2%) | His: 2 | (0.9%) | Ile: 18 | (8.2%) |
| Lys: 22 | (10.0%) | Leu: 14 | (6.4%) | Met: 10 | (4.6%) | Asn: 7 | (3.2%) |
| Pro: 7 | ( 3.2%) | Gln: 5 | (2.3%) | Arg: 7 | (3.2%) | Ser: 8 | (3.7%) |
| Thr: 14 | ( 6.4%) | Val: 14 | (6.4%) | Trp: 2 | (0.9%) | Tyr: 2 | (0.9%) |

### Lu22

Die Luziferase Lu22 ist ein Protein mit einem Molekulargewicht von 20,2 kDa und einem isoelektrischem Punkt von 7,89. Die kodierende Nukleotidsequenz ist: daraus ergibt sich eine Aminosäuresequenz von: und eine Aminosäurezusammensetzung von:

| | | | | | | | |
|---|---|---|---|---|---|---|---|
| Ala: 21 | (11.1%) | Cys: 11 | ( 5.8%) | Asp: 12 | (6.3%) | Glu: 9 | (4.7%) |
| Phe: 7 | ( 3.7%) | Gly: 21 | (11.1%) | His: 6 | (3.2%) | Ile: 13 | (6.8%) |
| Lys: 16 | ( 8.4%) | Leu: 12 | ( 6.3%) | Met: 7 | (3.7%) | Asn: | 4 (2.1%) |
| Pro: 6 | ( 3.2%) | Gln: 9 | ( 4.7%) | Arg: 6 | (3.2%) | Ser: 9 | (4.7%) |
| Thr: 6 | ( 3.2%) | Val: 13 | ( 6.8%) | Trp: 1 | (0.5%) | Tyr: 1 | (0.5%) |

Diese Sequenzen finden sich im Sequenzlisting wieder.

### Enzymatische Aktivität und biochemische Charakterisierung der Luziferasen

Die Proteine LuAL, Lu164 und Lu22 sind Enzyme, die unter Umsatz von Coelenterazin Licht freisetzen. Die gehören daher zu den Luziferasen. Die Luziferasen sind sowohl bakteriell als auch in eukaryotischen Zellen aktiv expremierbar. Die in eukaryotischen Zellen expremierten Luziferasen LuA1, Lu164 und Lu22 werden sekretiert. Bei der bakteriellen Expression erfolgt keine Sekretion.

Die Aktivität der Luziferasen ist temperaturabhängig. Für die Luziferasen LuAL und Lu164 konnten Temperaturoptima von 22°C (für LuAL) bzw. 27°C (für Lu164) bestimmt werden. Die Aktivität der Luziferase Lu22 hat ein Temperaturoptimum von 4°C oder niedrigeren Temperaturen.

### Beispiele

### Plasmide / Konstrukte

Als Vektoren zur Herstellung der im folgenden dargestellten Konstrukte wurden die Vektoren pcDNA3.1(+) und pTriplEx2 der Firma Clontech, sowie der Vektor pASMplr (Eigenkonstrukt mit cAMP sensitiven Promotorelementen; cre) verwendet. Die Derivate der Vektoren wurden als pcDNA3-x, pTriplEx2-x und pASM-x bezeichnet

### LuAL

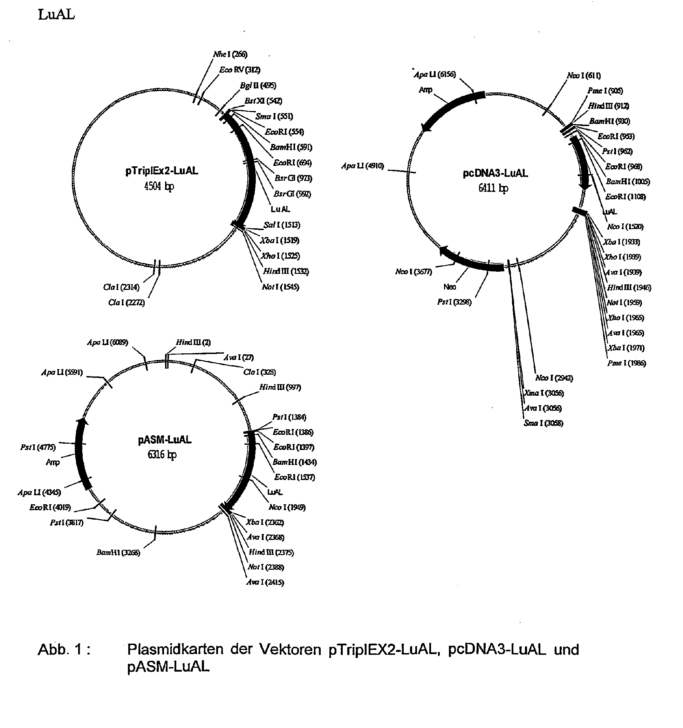

Die Abb. 1 zeigt die Plasmidkarten der Vektoren pTriplEX2-LuAL, pcDNA3-LuAL und pASM-LuAL

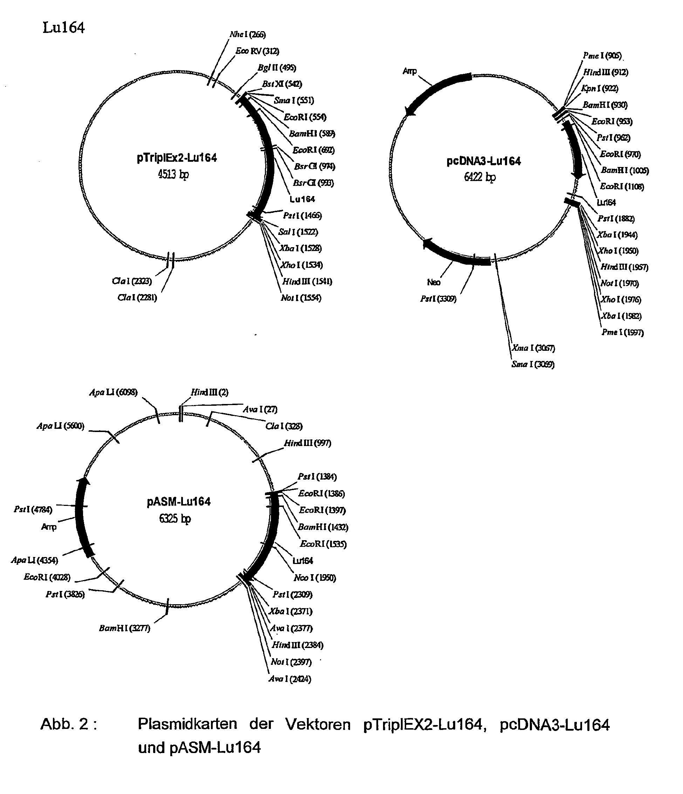

Die Abb. 2 zeigt die Plasmidkarten der Vektoren pTriplEX2-Lu164, pcDNA3-Lu164 und pASM-Lu164

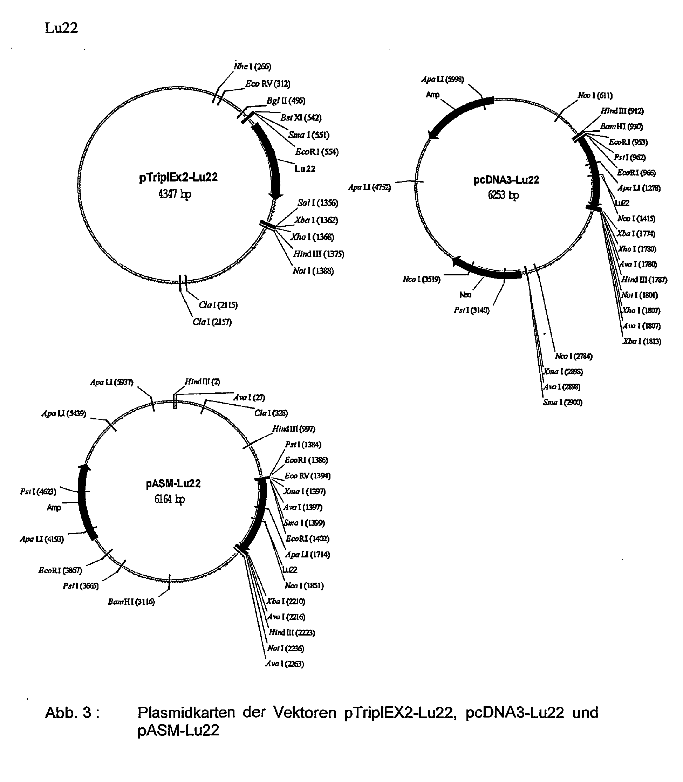

Die Abb. 3 zeigt die Plasmidkarten der Vektoren pTriplEX2-Lu22, pcDNA3-Lu22 und pASM-Lu22

### Coelenterazinderivate als Substrate von Lu164

Zur Identifizierung von Substraten für Lu164 wurden 10 µl Lösungen verschiedener Coelenterazinderivate (10⁻⁴ M) mit jeweils 10 µl Überstand von CHO-pcDNA3-Lu164 Zelllinien inkubiert und die Lumineszenz gemessen. Die Coelenterazine wurden von Molecular Probes (USA) bezogen.
Für die Luziferasen LuAL und Lu22 ergaben sich keine Unterschiede im Vergleich zur Luciferase Lu164. Als optimales Substrat für Lu164, LuAl und Lu22 konnte das nicht-modifizierte Colenterazin (Abb. B, Coelenterazin a)identifiziert werden.

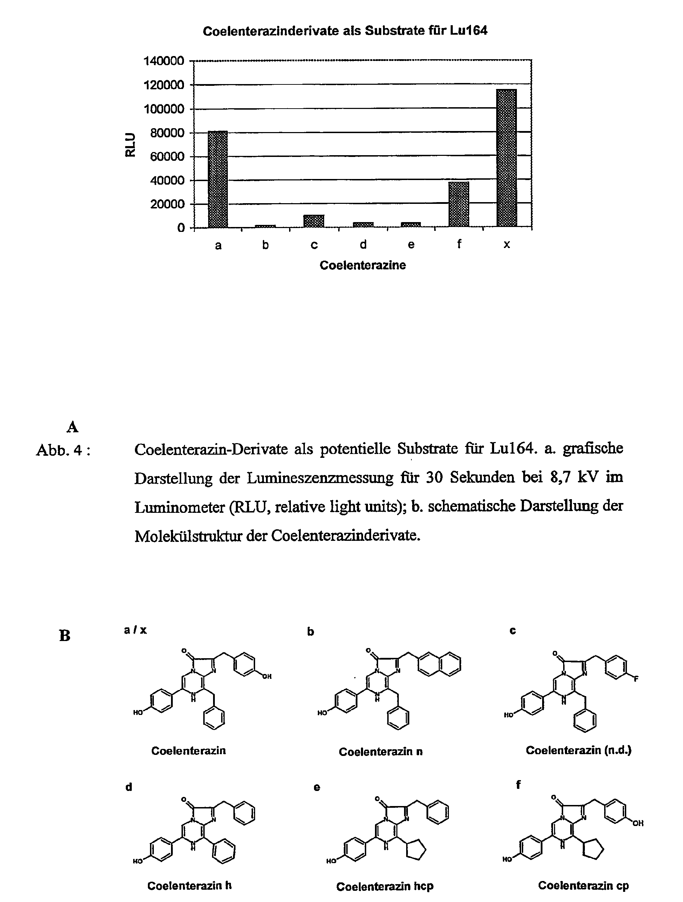

Die Abb. 4 zeigt Coelenterazin-Derivate als potentielle Substrate für Lu164 und eine grafische Darstellung der Lumineszenzmessung für 30 Sekunden bei 8,7 kV im Luminometer (RLU, relative light units); sowie schematische Darstellung der Molekülstruktur der Coelenterazinderivate.

### Enzymatische Aktivität der Luziferasen Lu164, LuAL und Lu22 in Abhängigkeit von Coelenterazin

### Bakterielle Expression

Die bakterielle Expression erfolgte im E. coli Stamm BL21(DE3) durch Transformation der Bakterien mit den Expressionsplasmiden pTriplEX2-Lu164, pTriplEX2-LuAL und pTriplEX2-Lu22. Die transformierten Bakterien wurden in LB-Medium bei 37°C für 3 Stunden inkubiert und die Expression für 4 Stunden durch Zugabe von IPTG bis zu einer Endkonzentration von 1 mM induziert. Die induzierten Bakterien wurden durch Zentrifugation geerntet, in PBS resuspendiert und durch Ultraschall aufgeschlossen. 5 µl des Lysates (5 mg/ml) wurde mit Coelenterazin (10⁻⁴ M in Methanol) oder Luziferin (Firefly-Luciferin) versetzt und die Chemolumineszenz gemessen.

Die Messung erfolgte bei 9,5 kV für 30 Sekunden in RLU (relative light units). Für Lu164 wurden 230000, für LuAL 320000 und für Lu22 260000 RLU gemessen. Die Expression erfolgte in E. coli BL21(DE3) mit den Vektoren pTriplEx2-Lu164, pTriplEx2-LuAL und pTriplEx2-Lu22.

### Eukaryotische Expression

Die konsütutive eukaryotische Expression erfolgte in CHO-Zellen durch Transfektion der Zellen mit den Expressionsplasmiden pcDNA3-Lu164, pcDNA3-LuAL und pcDNA3-Lu22 in transienten Experimenten. Hierzu wurden 10000 Zellen pro Loch in DMEM-F12 Medium auf 96 Loch Mikrotiterplatten plattiert und über Nacht bei 37°C inkubiert. Die Transfektion erfolgte mit Hilfe des Fugene 6 Kits (Roche) nach Herstellerangaben. Die transfizierten Zellen wurden über Nacht bei 37°C in DMEM-F12 Medium inkubiert. Die Messung der Chemolumineszenz im Mediums (5 µl) und Zelllysat (5 µl) erfolgte nach Zugabe von Coelenterazin (10⁻⁴ M in Methanol) für 30 Sekunden bei 9,5 kV im Luminometer bei Raumtemperatur.

Für Lu164 wurden 680000, für LuAL 670000 und für Lu22 510000 RLU (relative light units) gemessen. Die Expression erfolgte in CHO Zellen mit den Vektoren pcDNA3-Lu164, pcDNA3-LuAL und pcDNA3-Lu22.

### Emissionsspektrum der Luziferasen Lu164, LuAL und Lu22

Zur Messung des Emissionsspektrums wurden E. coli BL21(DE3) mit den Plasmiden pTriplEx2-Lu164, pTriplEx2-LuAL und pTriplEx2-Lu22 transformiert und überexpremiert, wie unter 3.1 beschrieben. 100 µl der Bakterienlysate wurden mit 50 µl Coelenterazin (10⁻⁴ M) versetzt und das Emissionsspektrum gemessen. Im Folgenden sind die Emissionsspektren der Luziferasen grafisch dargestellt.

Die maximale Emission durch den Substratumsatz erfolgt bei den Luziferasen LuAL, Lu164 und Lu22 bei einer Wellenlänge von etwa 490 nm.

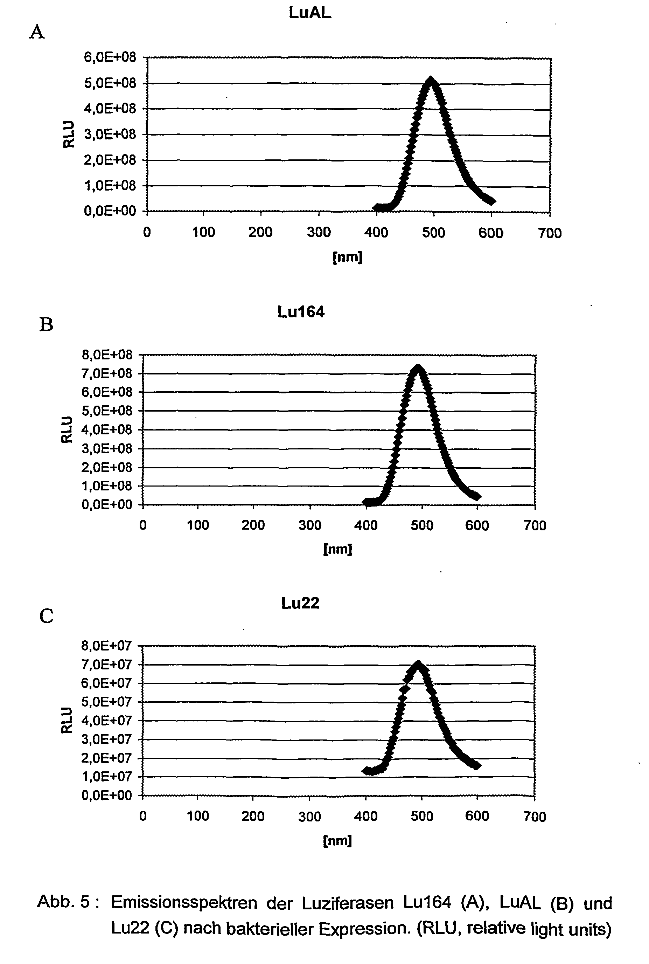

Die Abb. 5 zeigt Emissionsspektren der Luziferasen Lu164 (A), LuAL (B) und Lu22 (C) nach bakterieller Expression. (RLU, relative light units)

### Sekretion der Luziferasen Lu164, LuAL und Lu22 aus CHO-Zellen am Beispiel Lu164 und LuAL

Zur Charakterisierung der Expression der Luziferasen LuAl, Lu164 und Lu22 in eukaryotischen Zellen wurden CHO-Zellen mit den Plasmiden pcDNA3-LuA1, pcDNA3-Lu164, pcDNA3-Fireluc und pcDNA3.1(+) stabil transfiziert. Die erhaltenen Klone wurden in DMEM-F12 Medium kultiviert. Die Firefly Luziferase wurde als Positivkontrolle für eine nicht-sekretierte Luziferase verwendet Das Plasmid pcDNA3.1(+) diente als Kontrollplasmid zum Nachweis einer potentiellen endogenen Aktivität der CHO-Parentalzelle.

Zum Nachweis der Sekretion der Luziferasen wurden 2000 Zellen auf 384-Loch-Mikrotiterplatten plattiert. Nach 24 Stunden wurde das Medium entfernt, die Zellen mit Tyrode-Lösung gewaschen und 30 µl frisches Medium zugegeben. Anschließend erfolgte die erste Messung (0 h) nach der Zugabe von 5 µl Coelenterazin (10⁻⁴ M) bzw. Luziferin für die Firefly Luziferase im Luminometer bei 9,5 kV für 30 Sekunden. Nach ein bis fünf Stunden erfolgten die Messungen 1 h bis 5 h.

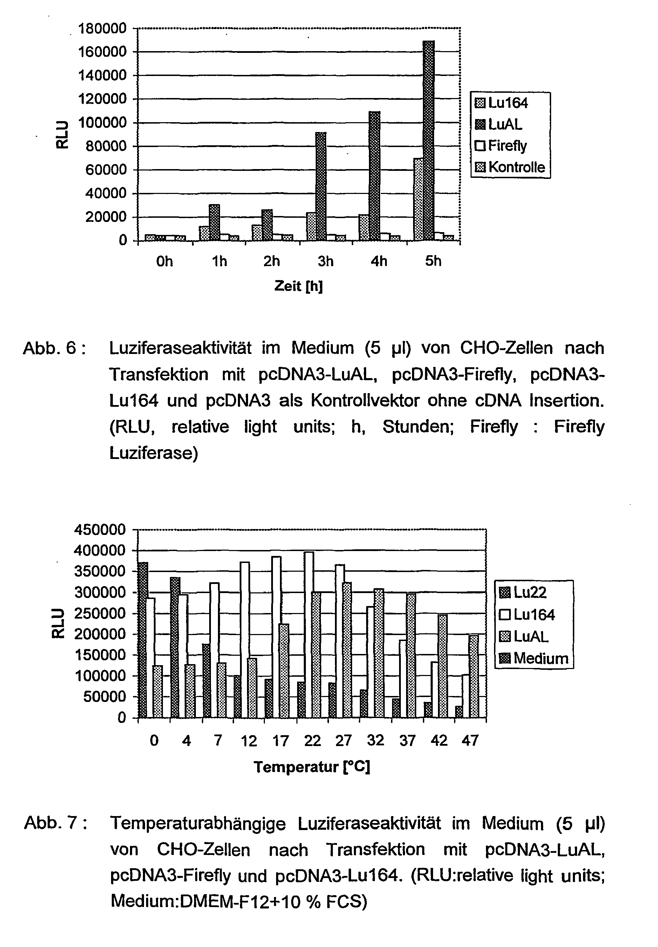

In Abbildung 6 ist die Zunahme der Luziferaseaktivität im Medium in Abhängigkeit von der Zeit dargestellt. Eine Sekretion der der Firefly Luziferase erfolgte nicht. Die Luziferasen LuAL, Lu164 nd Lu22 sind sekretorische Luziferasen.

Die Abb. 6 zeigt Luziferaseaktivität im Medium (5 µl) von CHO-Zellen nach Transfektion mit pcDNA3-LuAL, pcDNA3-Firefly, pcDNA3-Lu164 und pcDNA3 als Kontrollvektor ohne cDNA Insertion. (RLU, relative light units; h, Stunden; Firefly : Firefly Luziferase)

### Temperaturabhängigkeit der Luziferaseaktivität

Zur Bestimmung der Temperaturabhängigkeit der Luciferasen Lu22, Lu164 und LuAL wurden CHO Zellen transient mit den Vektoren pcDNA3-Lu22, pcDNA3-Lu164 und pcDNA3-LuAl transfiziert und die Luziferaseaktivität der Überstände bei Temperaturen zwischen 0 und 47°C bestimmt. Hierzu wurde der Zellüberstand, sowie die Coelenterazinlösung für 5 Minuten an die Messtemperatur adaptiert. Die Messung erfolgte für 30 Sekunden bei 9,5 kV im Luminometer.

In Abbildung 7 ist die gemessenen Lumineszenz in Abhängigkeit von der Temperatur für die Luziferasen LuAl, Lu164 und Lu22 dargestellt. Die Luziferaseaktivität von LuAL weist ein Temperaturoptimum von 27°C. Für Lu164 wurde ein Temperaturoptimum von 22°C und für Lu22 ein Temperaturoptimum von 4°C oder niedrigeren Temperaturen bestimmt.

Die Abb. 7 zeigt temperaturabhängige Luziferaseaktivität im Medium (5 µl) von CHO-Zellen nach Transfektion mit pcDNA3-LuAL, pcDNA3-Firefly und pcDNA3-Lu164. (RLU : relative light units; Medium: DMEM-F12+10 % FCS)

### Induzierte Expression der Luziferasen Lu164, LuAL und Lu22 in CHO-Zellen am Beispiel LuAL

Die induzierte eukaryotische Expression erfolgte in CHO-Zellen durch Transfektion der Zellen mit dem Expressionsplasmid pASM-LuAL in transienten Experimenten. Hierzu wurden 10000 Zellen pro Loch in DMEM-F12 Medium auf 96 Loch Mikrotiterplatten plattiert und über Nacht bei 37°C inkubiert. Die Transfektion erfolgte mit Hilfe des Fugene 6 Kits (Roche) nach Herstellerangaben. Die transfizierten Zellen wurden über Nacht bei 37 °C in DMEM-F12 Medium inkubiert. Die Induktion erfolgte für 5 Stunden durch Forkolin (10⁻⁵ M). Die Messung der Chemolumineszenz im Mediums und Zelllysat erfolgte nach Zugabe von Coelenterazin (10⁻⁴ M in Methanol) für 30 Sekunden bei 9,5 kV im Luminometer.

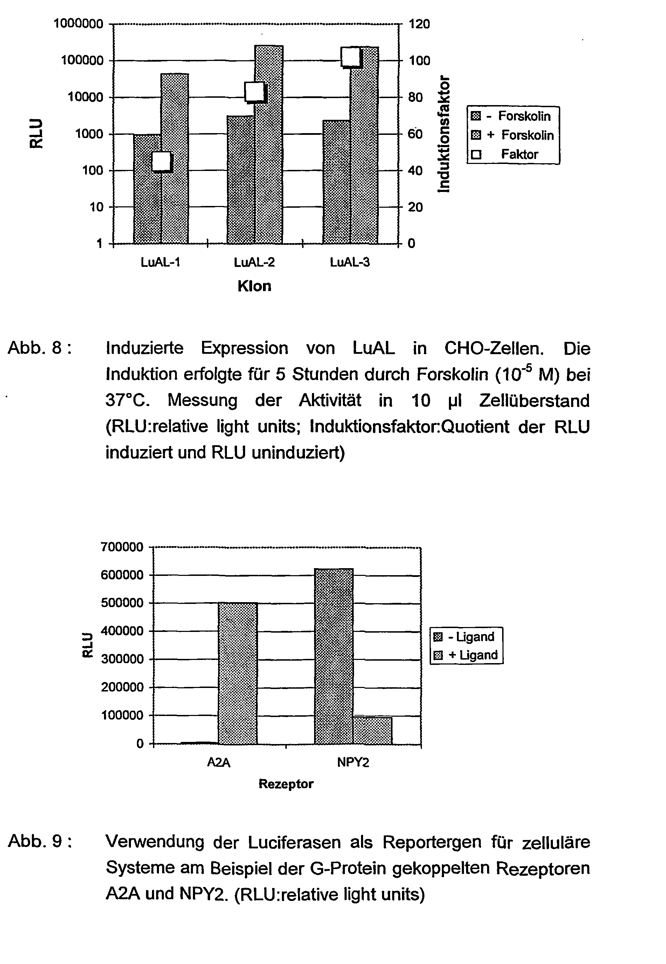

Die Abb. 8 zeigt die induzierte Expression von LuAL in CHO-Zellen. Die Induktion erfolgte für 5 Stunden durch Forskolin (10⁻⁵ M) bei 37°C. Messung der Aktivität in 10 µl Zellüberstand (RLU : relative light units; Induktionsfaktor : Quotient der RLU induziert und RLU uninduziert)

### Verwendung der Luziferasen Lu164, LuAL und Lu22 als Reportergen in zellulären Systemen am Beispiel der Rezeptoren NPY2 und A2A mit LuAL als Reportergen

Um die Aktivierung von G-Protein gekoppelten Rezeptoren durch Rezeptor-spezifische Ligand in Zell basierenden Systemen analysieren zu können, wurde die cDNA-Sequenz der Luziferase LuAL in den Expressionsvektor pASMplr kloniert. Der Expressionsvektor pASMplr enthält cAMP sensitive Promotorelemente (CRE), die eine indirekte Messung der intrazellulären cAMP Konzentration ermöglichen. Die Luziferase dient in dem System als Reportergen.

Die Verwendung der Luziferasen Lu22, Lu164 und Lu22 als Reportergene in zellulären Systemen wurde beispielhaft für die G-Protein gekoppelten Rezeptoren NPY2 (Neuropeptid Rezeptor 2) und A2A (Adenosin Rezeptor 2a) gezeigt. Hierzu wurde der stabile Klon CHO-pASM-LuAL mit dem Vektor pcDNA3-NPY2 bzw. pcDNA3-A2A transient transfiziert. Bei dem Rezeptor NPY2 handelt es sich um einen Gi, bei dem A2A Rezeptor um einen Gs gekoppelten Rezeptor.

Die Aktivierung des A2A-Rezeptors erfolgte für 4 h durch Zugabe von 1 µM NECA. Die Aktivierung s des NPY2 Rezeptors erfolgte durch Zugabe von 10 µM NPY2-Peptid in Anwesentheit von 10⁻⁵ M Forskolin. Die Messung der Luziferaseaktivität im Medium (30 µl) erfolgte nach Zugabe von Coelenterazin (10⁻⁴ M) für 30 Sekunden bei 9,5 kV im Luminometer.

Die Abb. 9 zeigt die Verwendung der Luziferasen als Reportergen für zelluläre Systeme am Beispiel der G-Protein gekoppelten Rezeptoren A2A und NPY2. (RLU : relative light units)

### Literatur / Patente

**Apley EC, Wagner R, Engelbrecht S.**, Rapid procedure for the preparation of ferredoxin-NADP+ oxidoreductase in molecularly pure form at 36 kDa., *Anal Biochem*. 1985 Oct;150(1):145-54.
**Berestovskaya NG, Shaloiko LA, Gorokhovatsky AY, Bondar VS, Vysotski ES, Maximov JE, von Doehren H, Alakhov YB.** Cotranslational formation of active photoprotein obelin in a cell-free translation system: direct ultrahigh sensitive measure of the translation course. *Anal Biochem.* 1999 Mar 1;268(1):72-8.
**Bonin et al.** Photinus pyralis luciferase. *Gene.* 1994 141:75-77
**Alam J, Cook JL.** Reporter genes: application to the study of mammalian gene transcription. *Anal Biochem*. 1990 Aug 1;188(2):245-54
**Cullen Bryan R., Malim Michael H.,** Secreted placental alkaline phosphatase as a eukaryotic reporter gene. *Methods in Enzymology.* 216:362ff
**Hastings,** Cell Physiology : Source book, Sperelakis N. (ed.), Academic press, pp665-681, 1995
**Head JF, Inouye S, Teranishi K, Shimomura O.,** The crystal structure of the photoprotein aequorin at 2.3 A resolution. *Nature*. 2000 May 18;405(6784):372-6.
**Gould S., Suresh S.**, Firefly luciferase as a tool in molecular an dcell biology. *Anal. Biochem.* 1988, 161:501-507
**Idelgaufts H.,** Gentechnologie von A-Z. *VCH* Verlag, Weinheim, 1993
**Inouye S, Noguchi M, Sakaki Y, Takagi Y, Miyata T, Iwanaga S, Miyata T, Tsuji FI.** Cloning and sequence analysis of cDNA for the luminescent protein aequorin. *Proc Natl Acad Sci USA* 1985 May;82(10):3154-8
**Inouye, S., Kakoi, H., Goto, T.,** Tetrahedron Lett., 34, 2971-2974 (1976)
**Inouye, S., Watanebe, H., Nakamura, H. and Shimomura, O.,** FEBS letters 481 (2000) 19-25
**Jones Keith, Hibbert Frank, Keenan Martine.** Glowing jellyfish, luminescence and a molecule called coelenterazine. *Tibtech.* 1999. 17: 477ff
**Kendall Jonathan M., Badminton Michael N.** Aequoria victoria bioluminescence moves into an exciting new era. *Tibtech.* 1998 16:216
**Krieg, S., Castles, C., Allred, D., Benedix, M., Fuqua S.,** RNA from air-dried frozen sections for RT-PCR and differential display. *Biotechniques.* 1996 Sep;21(3):425-8.
**Lorenz WW, McCann RO, Longiaru M, Cormier MJ.**, Isolation and expression of a cDNA encoding Renilla reniformis luciferase. *Proc Natl Acad Sci U S A*. 1991 May 15;88(10):4438-42.
**Lorenz WW, Cormier MJ, O'Kane DJ, Hua D, Escher AA, Szalay AA.,** Expression of the Renilla reniformis luciferase gene in mammalian cells. *J Biolumin Chemilumin*. 1996 Jan-Feb;11(1):31-7.
**Mathews J. C. et al.** Purification and propeties of rernilla reniformis luciferase. *Biochemistry*. 1977, 16(1): 85-91
**Matveev SV, Lewis JC, Daunert S**., Genetically engineered obelin as a bioluminescent label in an assay for a peptide. *Anal Biochem.* 1999 May 15;270(1):69-74.
**Phillips GN.** Structure and dynamics of green fluorescent protein. *Curr Opin Struct Biol.* 1997 Dec;7(6):821-7
**Sala-Newby et al.,** Expression of recombinant firefly luciferase. *Biochem Soc. Transact.* 1992, 20:143S
**Shimomura O, Johnson FH.,** Properties of the bioluminescent protein aequorin. *Biochemistry.* 1969 Oct;8(10):3991-7.
**Shimomura O.,** Bioluminescence in the sea: photoprotein systems. *Symp Soc Exp Biol.* 1985;39:351-72.
**Shimomura O, Teranishi K.,** Light-emitters involved in the luminescence of coelenterazine. *Luminescence.* 2000 Jan-Feb;15(1):51-8.
**Snowdowne KW, Borle AB.** Measurement of cytosolic free calcium in mammalian cells with aequorin. *Am JPhysiol.* 1984 Nov;247(5 Pt 1):C396-408.
**Thompson EM, Nagata S, Tsuji FI.,** Cloning and expression of cDNA for the luciferase from the marine ostracod Vargula hilgendorfii. *Proc Natl Acad Sci U S A.* 1989 Sep;86(17):6567-71.
**Thompson EM, Nagata S, Tsuji FI.,** Vargula hilgendorfii luciferase: a secreted reporter enzyme for monitoring gene expression in mammalian cells. *Gene.* 1990 Dec 15;96(2):257-62.
**Ungrin MD, Singh LM, Stocco R, Sas DE, Abramovitz M**., An automated aequorin luminescence-based functional calcium assay for G-protein-coupled *receptors. Anal Biochem.* 1999 Jul 15;272(1):34-42.
**Webster JJ, Chang JC, Manley ER, Spivey HO, Leach FR.,** Buffer effects on ATP analysis by firefly luciferase. *Anal Biochem.* 1980 Jul 15;106(1):7-11.
**Yang Te-Tuan, Sinai Parisa, Kitts Paul A. Kain Seven R.,** Quantification of gene expresssion with a secreted alkaline phosphatase reporter system. *Biotechnique.* 1997 23(6) 1110ff
**JP 05064583,** *Tora*, (TORAY IND Inc.), Luciferase modified with antigen, antibody, hapten or hormone, etc. - is isolated from Vargula hilgendorfii, useful in bioluminescent analysis
**JP 08027200,** *Tora,* (TORAY IND Inc.), Protein comprising luciferase fused to IgG binding domain - useful as diagnostic reagent in chemiluminescent immunoassays
**JP 09187281,** Kikk, (KIKKOMAN Corp.), Antibody-firefly luciferase fused protein - and related products i.e. firefly luciferase fused gene, recombinant DNA and its preparation
**US 5196524,** *Gustafson G.; Ingolia T.; Kirchner G.; Roberts J*., (Lilly & Co), Recombinant DNA encoding fusion protein with bacterial luciferase activity - comprises coding regions of lux A and B genes isolated from specific naturally bio luminescent bacteria, and DNA linker of specified restriction enzyme recognition site
**US 5418155,** *Milton J., Cornier; William W. Lorenz,* Isolated Renilla Luciferase and Method of use thereof
**US 5422266,** *Cormier M. J.; Prasher D.,* (UNIV GEORGIA RES FOUND INC), DNA coding for apo:aequorin - useful for recombinant prodn. of apo:aequorin which is a bio:luminescent marker in chemical and biochemical assays
**US 5670356,** *Sherf B.; Wood K* (Promega Corp), Modified firefly luciferase gene - encoding cytoplasmic form of luciferase enzyme
**US 5798441** *Cormier M. J.; Prasher D.,* (UNIV GEORGIA RES FOUND INC), New isolated apoaquorin polypeptide - useful as a luminescent marker in bio/chemical assays
**WO 0020619,** *Alan P.; Liu Jingxue,* Secreted renilla luciferase
**WO 9520653,** *Renard J P; Thompson E. M.; Thompson E.,* (INRA INST NAT RECH AGRONOMIQUE), Detecting transgenic embryos using Vargula luciferase gene as a marker - allows identification of such embryos at the blastocyst stage, also transgenic animals, embryos and cell line contg. this gene
**WO 9938999,** *Roelant C*. (Packard Instr. Co. Inc.), Detecting the presence of renilla luciferase in a sample by detecting luminescence of the sample

## Patentansprüche

1. Ein Nukleinsäuremolekül, ausgewählt aus der Gruppe der Nukleinsäuremoleküle umfassend:
a) ein Nukleinsäuremolekül, welches die in SEQ ID NO:2, SEQ ID NO:4, oder SEQ ID NO:6 gezeigte Peptidsequenz kodiert.
b) Ein Nukleinsäuremolekül, welches eine Peptidsequenz kodiert, die mindestens 90% Identität zu einer der Peptidsequenzen gezeigt in SEQ ID NO:2, SEQ ID NO:4, oder SEQ ID NO:6 aufweist, wobei es sich bei den Peptiden um sekretierte Luziferasen handelt.
c) Ein Nukleinsäuremolekül, umfassend ein Nukleinsäuremolekül wie dargestellt in SEQ ID NO:1, SEQ ID NO:3, SEQ ID NO:5, SEQ ID NO:7, SEQ ID NO: 8, SEQ ID NO:9, oder SEQ ID NO: 10, wobei die Nukleinsäuren Luziferasen kodieren.

2. Moleküle nach Anspruch 1, bei denen die Sequenz einen funktionalen Promotor 5' zur Sequenz enthält.

3. Rekombinanter RNA oder DNA Vektor, enthaltend ein Molekül gemäß Anspruch 2.

4. Einen nicht-humanen Organismus, enthaltend einen Vektor gemäß Anspruch 3.

5. Peptide, die durch die Nukleotidsequenzen nach Anspruch 1 kodiert sind.

6. Verwendung von Luziferasen gemäß den Ansprüchen 1 - 5 als Reportergene für zelluläre Systeme.

## Claims

1. A nucleic acid molecule selected from the group of nucleic acid molecules including:
a) a nucleic acid molecule which encodes the peptide sequence shown in SEQ ID No: 2, SEQ ID No: 4, or SEQ ID No: 6.
b) a nucleic acid molecule which encodes a peptide sequence which exhibits at least 90% identity with one of the peptide sequences shown in SEQ ID No: 2, SEQ ID No: 4, or SEQ ID No: 6, where the peptides are secreted luciferases.
c) a nucleic acid molecule comprising a nucleic acid molecule as depicted in SEQ ID No: 1, SEQ ID No: 3, SEQ ID No: 5, SEQ ID No: 7, SEQ ID No: 8, SEQ ID No: 9, or SEQ ID No: 10, where the nucleic acids encode luciferases.

2. Molecules according to Claim 1, in which the sequence comprises a functional promoter 5' in relation to the sequence.

3. Recombinant RNA or DNA vector comprising a molecule according to Claim 2.

4. A non-human organism comprising a vector according to Claim 3.

5. Peptides encoded by the nucleotide sequences according to Claim 1.

6. Use of luciferases according to Claims 1-5 as reporter genes for cellular systems.

## Revendications

1. Molécule d'acide nucléique, choisie parmi le groupe des molécules d'acide nucléique, comprenant :
a) une molécule d'acide nucléique, qui code la séquence peptidique montrée en SEQ ID N°2, SEQ ID N°4 ou SEQ ID N°6 ;
b) une molécule d'acide nucléique, qui code une séquence peptidique, qui présente au moins 90% d'identité avec une des séquences peptidiques montrées en SEQ ID N°2, SEQ ID N°4 ou SEQ ID N°6, où les peptides consistent en des luciférases sécrétées ;
c) une molécule d'acide nucléique, comprenant une molécule d'acide nucléique telle que représentée en SEQ ID N°1, SEQ ID N°3, SEQ ID N°5, SEQ ID N°7, SEQ ID N°8, SEQ ID N°9 ou SEQ ID N°10, où les acides nucléiques codent des luciférases.

2. Molécules suivant la revendication 1, dans lesquelles la séquence contient un promoteur fonctionnel en 5' de la séquence.

3. Vecteur à ARN ou à ADN recombiné, contenant une molécule suivant la revendication 2.

4. Organisme non humain, contenant un vecteur suivant la revendication 3.

5. Peptides, qui sont codés par la séquence des nucléotides suivant la revendication 1.

6. Utilisation de luciférases suivant les revendications 1 à 5, comme gène reporter pour systèmes cellulaires.
